# EUROPEAN PATENT APPLICATION

(11) **EP 2 111 792 A1**
(43) Date of publication of application: **28.10.2009**
(21) Application number: 09158458.1
(22) Date of filing: 22.04.2009
(51) Int. Cl.: A61B 5/12

(54) **REFLECTOMETER**

(30) Priority: 24.04.2008 FI 20080316
(71) Applicant: Oulun Seudun Ammattikorkeakoulu, 90250 Oulu (FI)
(72) Inventor: Hannula, Manne, 90440, Kempele (FI); Hinkula, Henry, 90550, Oulu (FI)

(57) **Abstract**

This publication describes an acoustic reflectometer, the function of which is distributed into separate parts so that it consists of a distinct measurement tip 1 which is connected to a personal computer 2, and where the measurement function and interpretation of the measurement result is executed with help of an internet page 3. The acoustic reflectometer according to the invention includes a function to verify the correct posture of the measurement tip, a function to guide the correct posture of the measurement tip and a function to analyze the measurement result, which makes it possible to essentially solve reliability problems and measurement signal interpretation problems related to the acoustic reflectometer. The embodiment of the invention is especially cost effective, which establishes wide scale use of the application in health care systems.

## Description

This invention concerns an acoustic reflectometer as described in the preamble of claim 1.

Acoustic reflectometers are devices which establish measurements for evaluation of properties of the tympanic membrane of the ear. The principle of the acoustic reflectometer is to input a specific sound stimulus to the outer auditory canal, to measure the reflected response signal, and then to analyse properties of the tympanic membrane on the basis of the response signal. The measurement can be utilized in diagnostics of otitis media. For example, if there is otitis media related fluid behind the tympanic membrane, this can be monitored from specific features of the response signal of the acoustic refletometry measurement.

The known acoustic reflectometers which are in commercial use are like the devices presented in publications US5699809 and US5594174. In those solutions the acoustic reflectometer measurement system is implemented as a handheld device including a measurement tip which is put to the entrance of outer auditory canal. The device includes a small speaker and a microphone, which establish producing of the sound stimulus and measurement of it to and from the outer auditory canal. The device is equipped with a microprocessor or the like electronic part which produces the sound signal to the speaker and records the signal from the microphone. The recorded signal is analysed with the electronic part which includes the analysis algorithm, the result of which the device shows on its display.

Further, publications EP1447048, US2006281975, US2007197881 and US2003065252 illustrate state of the art in field of acoustic reflectometry or distributed measurement technology and its applications.

The principles of the acoustic reflectometer are shown for example in publication US4601295. Typically the application idea of acoustic refletometry is to establish easy to use, comfortable and cheap means for analysis and diagnosis of otitis media, especially in children, and in this way, for example, to improve efficiency of health care system.

However, the known acoustic reflectometry solutions have several problems related to the measurement technique, especially concerning the analysis of the measured signal. In addition, cost effective implementation of the device has comprehensive problems, which make it difficult to apply the device as a support of health care system in a wide scale.

The measurement technique related problem of known acoustic reflectometers concerns the problems of measurement and analysis of the signal. To make a reliable measurement, the measurement tip should be in a correct posture in relation to the outer auditory canal. Furthermore, the interpretation of the measured signal is complicated, difficult, and in some cases even impossible. Many research reports have shown that the sensitivity and specificity values of the acoustic reflectometer remain clearly below one hundred percent for example in diagnostics of otitis media on the basis of the measurement result.

Furthermore, an application related problem with the known acoustic reflectometers is the fact that their commercial cost effective implementation is difficult because of the complexity of the device. Namely, the devices include many high-priced parts, for example measurement electronics, microprocessor, display, and so on. This prevents the wide scale utilization of the devices.

The object of the invention is to solve the problems associated with the known acoustic reflectometer solutions relating to the reliability of the measurement technique and to the interpretation of the measured signal. In addition, the invention makes it possible to implement an acoustic reflectometer in a manner which solves the problem about the cost effective implementation of it.

The object of the invention can be achieved with help of acoustic reflectometer which is implemented in a distributed manner and which consists of a measurement tip, a personal computer and an internet page. The measurement tip includes a small sized microphone and a speaker and is at its one end equipped with a funnel which can be put on the entrance of the outer auditory canal. The measurement tip includes a microphone and speaker cable provided at its one end with a conventional connector which is compatible with the microphone and headset connectors of the sound card of the personal computer. In addition, the personal computer is provided with an internet access. The internet page is a specific collection of www-pages including functions by means of which acoustic reflectometry measurement may be performed via the personal computer. The internet page includes specific functions which guide a person performing the acoustic reflectometry measurement in order to obtain a reliable measurement result. In addition, the internet page includes specific functions for analysis of the measured signal. The internet page may also have a continuous real time connection for example with a physician who may guide a user performing the acoustic reflectometry measurement, or who may interpret the measurement results obtained by means of the internet page.

More exactly, the acoustic reflectometer according to the invention is characterized in what is presented in the characterising part of claim 1.

Next the invention is described by means of some exemplary embodiments with reference to Figure 1 which presents one example of the embodiment of the acoustic reflectometer according to the invention, Figure 2 which presents a scheme of the functions relating to the invention, Figure 3 which illustrate structure of software used in the invention, and to Figure 4, which exemplifies application of invention for monitoring of development of otitis media status of the patient.

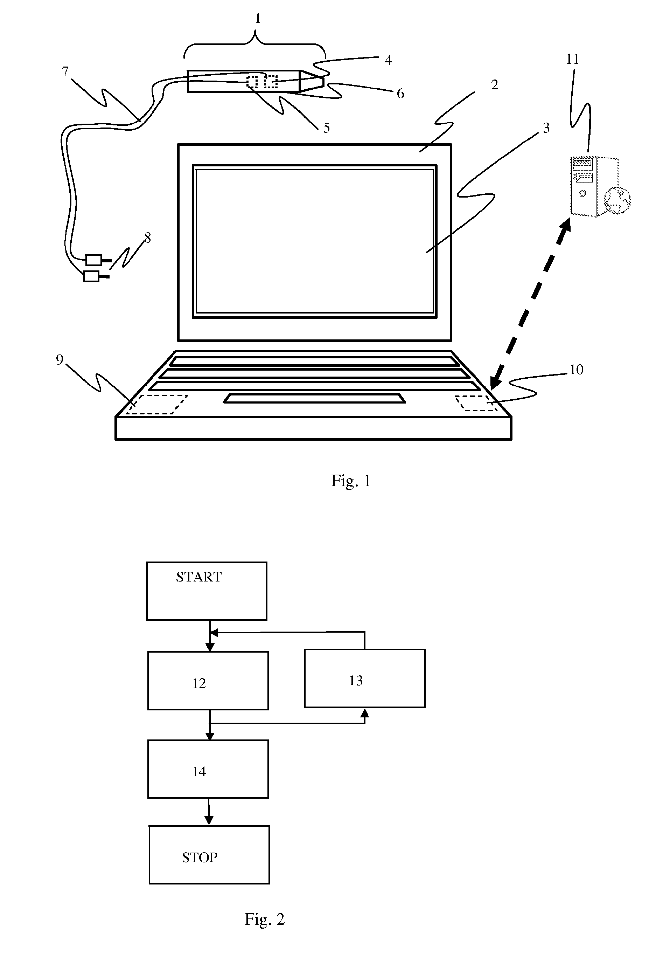

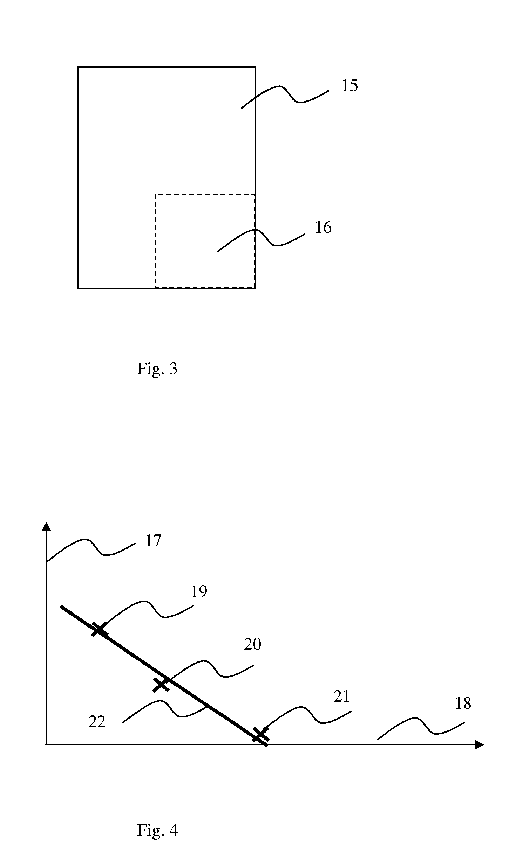

An acoustic reflectometer consists of a measurement tip 1, a personal computer 2 and an internet page 3. The measurement tip includes a speaker 4 and a microphone 5, enclosed to a plastic cover 6, which is designed to have in its other end a funnel which fits to the entrance of the outer auditory canal. The measurement tip has a cable 7 including connectors 8 to connect the speaker and the microphone in the measurement tip to the personal computer. The personal computer has a sound card 9 and means for internet access 10 which establish connection to the internet page 3 on a server 11, which internet-page can be seen on the screen of the personal computer 2. The internet page 3 is a www-page which has functions illustrated in Figure 2; the function to verify correct posture of the measurement tip 12, the function to guide the correct posture of the measurement tip 13 and the function to analyze the measurement result 14. The function 12 can be implemented for example as follows: first the amplitude spectrum of the measured signal is calculated. The measured amplitude spectrum is compared with a template locating in the software of the internet page 3, which illustrates a correct posture of the measurement tip, and if the measured amplitude spectrum deviates more than 10% of the template spectrum, the result of the function 12 is "incorrect posture" and otherwise the result is "correct posture". Further, the function 13 can be implemented as follows: if the result of the function 12 is "incorrect posture", the function 13 outputs "change slightly posture of the measurement tip according to the ear canal" to the screen of the personal computer 2, and in case the result of the function 12 is "correct posture", it outputs "posture is correct" to the screen of the personal computer 2. The internet page 3 is located in the server 11 and it is implemented with HTML-software entity 15, shown in Figure 3. It includes java -based software part 16 to establish use of sound card 9 of the personal computer 2 to execute measurements with the measurement tip 12.

The invention is used as follows: the user turns his personal computer 2 on and connects the connectors 8 in the cable 7 of the measurement tip 1 to the corresponding connectors of the sound card 9 of the personal computer 2. Next the user opens the internet page 3 with help of means for internet access 10 and a web browser. The internet page 3 installs software part 16 to the personal computer 2 to establish the measurements with the sound card 9. In the internet page the user clicks an icon which is named like "Start measurement". Next the internet page 3 begins to send sound signal to the user via www-connection and simultaneously it begins to measure it with help of the measurement tip 1 connected to the personal computer 2. During this operation the function for verifying the correct posture of the measurement tip 12 investigates on the basis of the measured signal if the measurement tip is correctly put to the entrance of the outer auditory canal. If the measurement tip is, on the basis of measured signal, for example too loosely put to the entrance of the outer auditory canal, the function to guide the correct posture of the measurement tip 13 prints to the internet page 3 instructions to put the measurement tip slightly more tightly to the entrance of the outer auditory canal. Correspondingly, if the measurement tip is, for example, at too large an angle to the outer auditory canal, the function to guide the correct posture of the measurement tip 13 shows corresponding instruction to correct the posture on the internet page 3. This process continues following the procedure illustrated in Figure 2, until the function to verify correct posture of the measurement tip 12 has detected that the measurement tip 1 is properly on the entrance of the outer auditory canal. After this phase the internet page informs the user that the measurement posture is correct, and next the measurement begins, during which the internet page 3 sends sound signals at different frequencies to the measurement tip and simultaneously records the corresponding responses. After this operation, the function to analyze the measurement result 14 analyzes with help of an appropriate algorithm the result, and shows the result for the user on the screen of the internet page 3. The function 14 may be executed for example as follows: first the amplitude spectrum of the measured signal is calculated. Then the resulting amplitude spectrum is compared with three different templates locating in the software of the internet page 3; first illustrating the amplitude spectrum of the acoustic reflectometry measurement signal from healthy ear, second illustrating corresponding amplitude spectrum of ear with moderate otitis media and third illustrating the spectrum of ear with severe otitis media. The function 14 results to "healthy", "moderate otitis media" or "severe otitis media", according to template, which has the smallest deviation with the amplitude spectrum of the measured signal. In another embodiment, the result may be, for example, an estimate of the presence of otitis media in the measured ear on the basis of the measurement result.

Within the scope of the invention even solutions deviating from the aforedescribed can be considered. For example, the speaker 4 in the measurement tip may be a stereo speaker with an arrangement where one speaker produces the sound signal needed in the acoustic reflectometry measurement and the other speaker is put to the outer cover of the measurement tip, so that the instructions of the function to guide the correct posture of the measurement tip 13 can be heard as voice instructions from it in order to set the measurement tip to the correct posture. Furthermore, the same speaker on the outer cover may be used for announcing the result of the function to analyze the measurement result 14 for the user as a voice message. Additionally, the internet page 3 relating to the invention may be implemented in many different manners. For example, the page may have means for selecting which features of the measurement should be interpreted, or which algorithm should be used for the interpretation. For example, the page may have, relating to the function to analyze the measurement result 14, three different algorithms, which has been done by different research institutions which all predict probability of otitis media on the basis of the measurement of the ear. Moreover, the page may have algorithm to assess if the current otitis media is getting better or deteriorating as exemplified in Figure 4, where the y-axis 17 shows the level of otitis media, x-axis 18 is time, 19 is first measurement, 20 is second measurement and 21 is third measurement done with one day intervals, and line 22 is line, fitted to the measurement points. The algorithm for assessing the status of the otitis media in time domain may be based on the slope value of this line; if it is negative, the algorithm output is "otitis media is getting better", and if it is positive, the output is "otitis media is detoriating". Furthermore, the page may include a possibility to select an option to ask oto-rhino-laryngology - physician or a corresponding professional to interpret the measurement result. This professional may be on duty behind the service, waiting for the like interpretation acquires. Furthermore, the measurement function can be implemented in an interactive form so that the person on duty behind the internet page 3 guides personally the measurement during the whole acoustic reflectometry measurement process. Moreover, the measurement solution can be implemented also so that the connectors 8 of the cable 7 of the measurement tip 1 are of form which is compatible with, in addition to a personal computer, also some other electronic device which is generally available, for example a mobile phone, palm computer or mp3 player. In case of the mobile phone the connection would be done together with the hands-free headset speaker-microphone connector. In this case the invention could be used via the mobile phone, in case it is equipped with an internet connection. Further, the invention can be implemented also so that the device to which the measurement tip 1 is connected will be equipped with a software which establishes measurement with the device also without internet connection, or in a manner where the interpretation of the measurement result in comprehensive level is possible only when the internet connection is available, or in a manner where with help of internet connection it is possible always, when needed, to install the latest and best stand-alone measurement and analysis software to it.

Some preferred embodiments of the invention are described above. The invention, however, is not limited to the previously described embodiments. The inventive idea can be applied in several ways within the limits of the claims.

## Claims

1. An acoustic reflectometer for evaluation of properties of the tympanic membrane, **characterised in that** its measurement function and interpretation of the measurement result is executed completely or partially with help of an internet page (3).

2. The acoustic reflectometer according to claim 1, **characterised in that** the internet page (3) includes a function to verify correct posture of the measurement tip (12) and a function to guide the correct posture of the measurement tip (13) in order to establish verification of correct posture of the measurement tip (1) on the entrance of the outer auditory canal for obtaining a reliable measurement result.

3. The acoustic reflectometer according to claim 1, **characterised in that** the function to analyse the measurement result (14) in the internet page (3) is equipped with different options in order to interpret specific selected characteristics from the measurement result.

4. The acoustic reflectometer according to claim 1, **characterised in that** the means to access the internet page (3) is personal computer (2) or some other information processing device, equipped with means for internet access, like a mobile phone, palm computer, mp3 player or some other electronic device which is generally available and to which the measurement tip (1) can easily be connected.
